Europäisches Patentamt

(19) European Patent Office

Office européen des brevets

(11) Publication number: **0 246 760 B1**

# (12) EUROPEAN PATENT SPECIFICATION

(45) Date of publication of patent specification: **08.07.92**   (51) Int. Cl.⁵: **G01N 33/53**, G01N 33/537, G01N 33/543, G01N 33/545

(21) Application number: **87303647.9**

(22) Date of filing: **24.04.87**

�External

(54) **Variable volume assay device and method.**

(30) Priority: **24.04.86 US 855730**

(43) Date of publication of application:
**25.11.87 Bulletin 87/48**

(45) Publication of the grant of the patent:
**08.07.92 Bulletin 92/28**

(84) Designated Contracting States:
**AT BE CH DE ES FR GB GR IT LI LU NL SE**

(56) References cited:
**EP-A- 93 613**
**DD-A- 231 130**

**WO 86/03589**

(73) Proprietor: **E-Y LABORATORIES, INC.**
**127 North Amphlett Boulevard**
**San Mateo California 94401(US)**

(72) Inventor: **Chu, Albert E.**
**140 Roblar Avenue**
**Hillsborough, CA 94010(US)**
Inventor: **Chun, Peter K.**
**2566 Adams Court**
**South San Francisco, CA 94080(US)**

(74) Representative: **Bizley, Richard Edward et al**
**BOULT, WADE & TENNANT 27 Furnival Street**
**London EC4A 1PO(GB)**

**Description**

This invention relates to a device and method for the testing of liquids and particularly samples derived from biological fluids, using immunological diagnostic techniques.

There are a wide variety of immunoassays in the prior art in which one component of an immunological pair, e.g., antigen or antibody, is detected or measured using labeled antigen or antibody. In one technique, known as the competitive binding technique, a competition is set up between the component to be detected and a labeled reagent of the same immunological type. For example, for the detection of antigen in a liquid sample, the sample and a labeled antigen are contacted with antibody specific for the antigen. The amount of labeled antigen which binds to the antibody is inversely proportional to the amount of antigen in the sample.

In another assay, known as a sandwich assay, immobilized capture antibody is bound to a solid surface and antigen in the sample immunologically reacts with that antibody. A second labeled antibody also reacts with the antigen to form an immobilized reaction product. The label in the reaction product is detected as an indication of the presence of the antigen in the sample.

The term "labeled" means that the immunological reagent is formed into a conjugate with a label or tag capable of emitting a signal or of causing the emission of a signal. Various labels include radioactive isotopes, chromogens, metal sol particles (specifically colloidal gold, silver and chelated gold), and enzymes. In each instance, these labels are detectable, either directly or indirectly. An enzyme label is reacted with a substrate which is converted to a visible signal (e.g., light or fluorecence) only in the presence of that enzyme.

For the detection or measurement of an antigen using a sandwich technique, polyclonal antiserum or antibodies withdrawn from an animal have been used for many years for both the labeled antibody and for the capture antibody on the surface. More recently, monoclonal antibodies have been used in place of the polyclonal antibodies in the assay. One such system is described in Wada et al., Clin. Chem., 28, No. 9, 1982, pp. 1862-1866, the solid phase antibody is directed to one subunit of a particular antigen, hCG, while an enzyme-coupled monoclonal antibody is directed to another subunit. In this sandwich assay, the inside of the test tube to which the sample is added is the solid surface on which the capture antibody is immobilized. Other solid surfaces include a dip stick, plastic ball or magnetic ball, in which the capture antibody is retained.

Reaction on a solid surface can be relatively slow because of the limited contact between the immobilized or soluble reagent and the sample component. The time for the assay has been reduced by immobilizing the capture antibody within a porous membrane so that the antibody molecules are exposed in a three-dimensional matrix. In such systems the liquid sample containing antigen passes through the membrane into an underlying absorbent material which draws the sample. One such system, disclosed for use in a competitive binding assay, is Bagshawe U.S. 3,888,629. Another system disclosed for use in a sandwich assay is disclosed in Valkris et al., Clin. Chem., Vol. 31, No. 9, 1985, pages 1427-1431. This system also uses a porous membrane through which the liquid sample travels into an absorbent bed. A further system in which an immunological reaction takes place in a membrane through which the reagents and liquid sample pass into an absorbent material to reduce the reaction time is disclosed in Tom et al., U.S. 4,366,241.

Another membrane system with an underlying absorbent material is disclosed in Cole et al., U.S. 4,246,339. Liquid sample is initially placed in an array of test wells having microporous membrane bottom walls at the top of the device. A reaction surface membrane with porous absorbent material is disposed below the array, with an intermediate chamber defined between them. The membranes of the test wells are maintained out of contact with the reaction surface membrane until the beginning of the reaction procedure at which time they contact each other and the liquid is drawn through both membranes into the absorbent material. A signal emitted by the reaction product is viewed on the reaction surface membrane. Air vents are provided for the intermediate chamber so that a change of volume does not effect the internal pressure.

All of the above membrane devices are "once-through" systems in that the liquid which is passed through the membrane is taken up by absorbent material and so cannot be passed in the opposite direction. Thus, if capture antibody is concentrated at a specific spot on the membrane, only the substance in an imaginary column directly above that point is seen by the antibody in that spot. This limits the degree of mixing and thus the sensitivity and speed of the system.

In one aspect the invention provides an assay device comprising a container defining a chamber of variable volume, means for varying the volume of the chamber, an inlet port in fluid communication with the chamber, and a means disposed across the fluid path through the inlet port, characterised in that the said means disposed across the fluid path comprises a fluid permeable membrane means, a reagent reactive with the sample component in a complementary binding assay is retained within the membrane means, and the chamber is essentially fluid sealed from the exterior of the container except through the membrane means, whereby a liquid sample on the membrane means outside of the chamber is drawn through the membrane means into the chamber by suction created upon actuation of the volume varying means to increase the volume of the chamber, and the liquid sample within the chamber is forced out of it under internal pressure created upon actuation of the volume varying means to decrease the volume of the chamber.

Preferably, the membrane means includes a membrane layer, on which an immunological reagent is immobilized and a porous support is positioned adjacent to the membrane. A further immunological reagent, such as a labeled immunological component, soluble in the liquid sample to be analyzed, may also be coated onto the porous support. The reagent is dissolved upon passage through the support of the liquid sample.

In another aspect the invention provides a method of detecting at least one predetermined component in a liquid sample, comprising

(a) contacting the liquid sample with one side of a fluid permeable membrane means disposed across an opening into a chamber otherwise substantially sealed from its surroundings, the chamber being of variable volume, and

(b) varying the chamber volume to move the liquid sample through the membrane means into or out of the chamber and again varying the volume of the chamber to move the liquid sample back through the membrane means, whereby the predetermined component may react with at least one reagent in a complementary binding assay to form a detectable reaction product on the membrane means as an indication that the component is present in the liquid sample.

In a sandwich assay embodiment, the liquid sample is biologically derived (e.g., urine or serum) and includes an antigen or antibody to be assayed.

For analysis of antigen, one reagent capture antibody is immobilized on the membrane to which the antigen binds.

Another reagent, soluble labeled antibody, reacts with the bound antigen to form a detectable reaction product on the membrane means.

In a preferred embodiment, the chamber volume is increased and decreased a number of times to cycle the sample back and forth through the membrane to improve the efficiency of mixing and thereby reduce the reaction time and also increase the sensitivity of the assay. To begin the cycle, the initial sample may either be deposited on the external surface of the membrane means or filled within the chamber, as desired.

In another preferred embodiment, the one reagent is concentrated on at least one defined region, such as a dot on the membrane and the detectable reaction product is formed at the dot. For example, referring to a sandwich assay for antigen, the capture antibody is retained at a dot on the membrane to capture the antigen which in turn immunologically reacts with the labeled antibody at the same place. Multiple dots using antibodies specifically reactive with different antigens may also be employed to simultaneously assay different antigens in the sample.

In the accompanying drawings:

Figure 1 is a top view of an assay device;

Figure 2 is a side view of the assay device of Figure 1;

Figures 3a and 3b are cross-sectional views taken along the line 3-3 of the device of Figure 1 in closed and open positions, respectively.

Figure 4 is a side view of another assay device;

Figures 5 and 6 are cross-sectional views taken along the lines 5-5 and 6-6 of Figure 4, respectively;

Figures 7 to 13 are cross-sectional views of other assay devices; and

Figure 14 is an enlarged view of the circled membrane portion of Figure 13.

The assay devices, as illustrated, will be described first followed by a description of some of the different assay techniques which can be employed using the device.

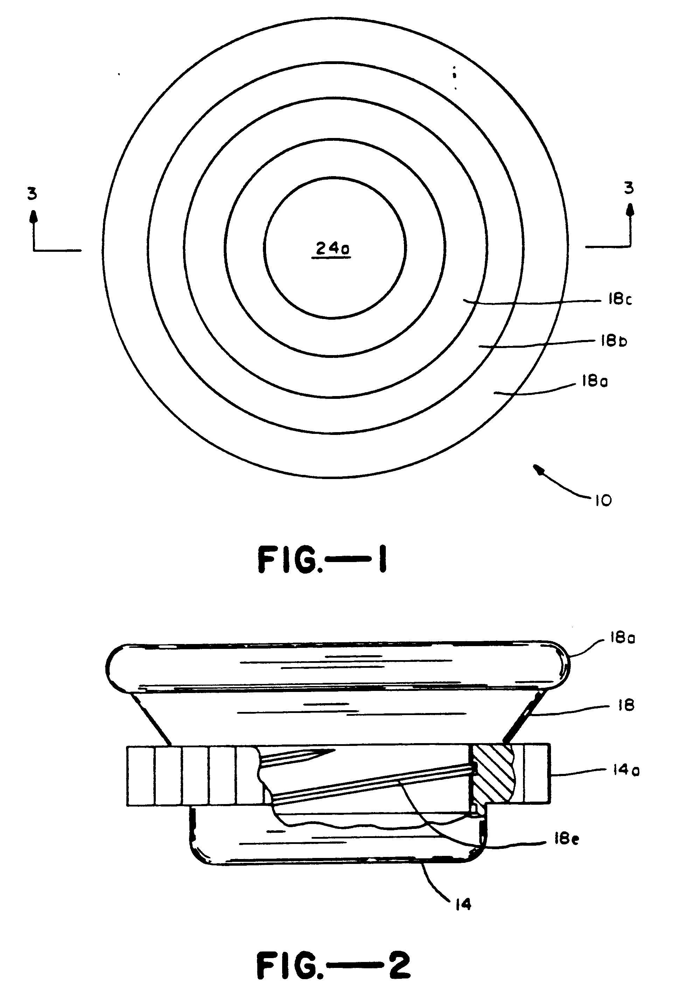

Referring to Figures 1 to 3 of the drawings, an assay device 10 is in the form of a container defining a hollow interior space or chamber 12 of variable volume. The container comprises a hollow body 14 and a piston 16 mounted for movement into and out of the body upon relative movement between the piston and body. The piston 16 serves as a movable wall forming a part of the container for varying the volume of chamber 12. As illustrated, the body 14 has a generally U-shaped vertical cross-section and a circular horizontal cross-section. A side wall 14a of the U defines a gripping surface in the form of a rim of generally rectangular cross-section disposed at the upper part of the arms of the U. The base portion of the U has a substantially longer diameter than the height of the arms. The inner wall of the body 14 includes a spiral groove 14b in which engages the threads of the piston 16 as described below. The body 14 is suitably formed of a plastic material such as polyethylene, polypropylene, polystyrene or polyvinyl chloride, or of a durable material such as a metal (e.g., aluminum) which permits reuse of the device 10, if desired.

Piston means 16 comprises a circular piston member 18 with an externally accessible gripping surface 18a. Piston 18 includes a circular upright wall portion 18b which, with body 14, defines chamber 12. Wall portion 18b forms a rim for the containment of liquid in chamber 12. Piston member 18 further includes an inwardly projecting circular ridge 18c serving to seat an inwardly projecting convex inner wall of adjacent annular retaining insert 20. Piston member 18 also includes an O ring run 18d in which is seated a pliable O ring 22, suitably formed of rubber. Piston member 18 also defines a spiral externally projecting thread 18e which is seated within spiral groove 14b of body 14.

Membrane means 24 is retained within the open center of piston member 18 by pressure fit within insert inner wall 20a. As illustrated, membrane means 24 includes a porous membrane in the form of a membrane disc 24a with an externally visible upper surface and an independent porous support disc 24b disposed adjacent and interior of membrane 24a, also retained by insert wall 20a.

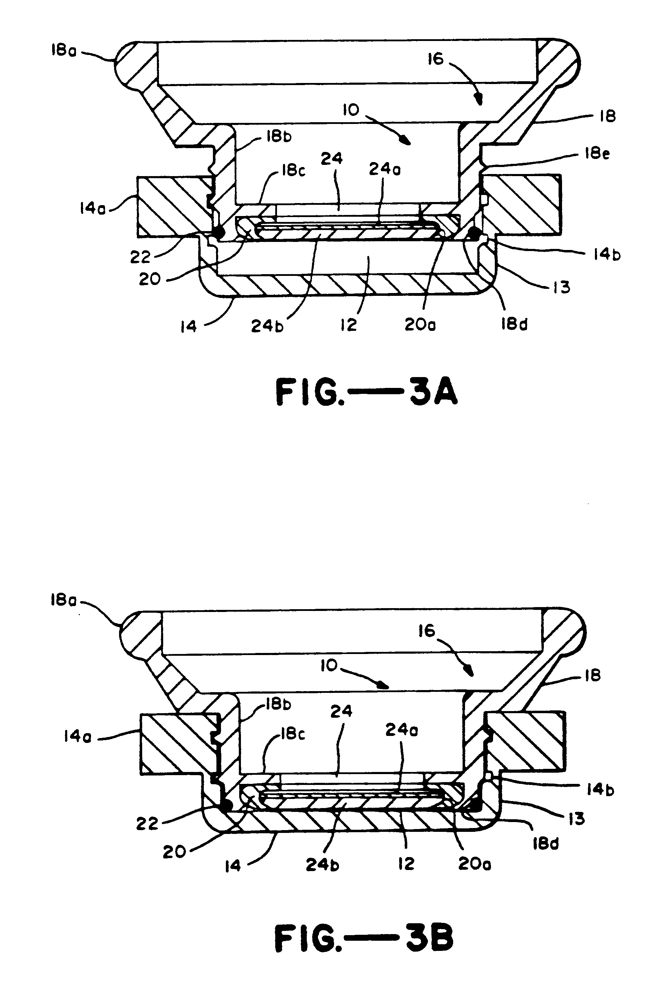

Referring to Fig. 3b, the device is illustrated with variable chamber 12 approximately at minimum volume. By gripping surfaces 14a and 18a, piston member 18 can be twisted relative to body 14 to cause the piston member to be moved outwardly from the body and to increase the volume of chamber 12. Chamber 12 is substantially sealed from the environment except through membrane means 24. O ring 22 serves to retain a pressure seal against the inner wall of the body when piston member 18 is moved. Thus, if liquid is placed on the external surface of membrane 24a with piston member 18 in the position illustrated in Fig. 3b and the piston member is twisted to increase chamber 12 to a position illustrated in Fig. 3a, the increased chamber volume causes the liquid to be drawn into the chamber by suction. Conversely, the liquid is passed in the opposite direction under internal pressure created upon movement of piston member 18 back to the position illustrated in Fig. 3b.

The membrane of membrane disc 24a may be of any type capable of immobilizing reaction product of the reagents and sample component without adversely affecting the reaction, and which permits passage, without undue pressure drop, of the remainder of the liquid sample or of a washing solution. A suitable membrane is formed of nitrocellulose or nylon, although other types of membranes with these characteristics can be employed. The porosity of the membrane preferably varies from about 0.2 to about 12 $\mu$m (microns).

As described below, in a typical system, an immunological reagent is first immobilized on the membrane. The reagent immunologically reacts with and captures the predetermined component of the liquid sample to be detected. Such immunological reagent, typically a protein such as an antibody or antigen, can be immobilized directly onto certain membranes, such as nitrocellulose, by absorption or by covalent bonding.

The depth or thickness of the membrane is selected so that an adequate amount of immunological reagent can be immobilized to capture the sample component. However, the thickness should not be so great as to cause undue pressure drops by the passage of the liquid sample through the membrane. For this purpose, a suitable thickness for the membrane is from 100 $\mu$m (microns) to 200 $\mu$m (microns).

Membranes of the above thickness when subjected to pressure forces applied by the present device may not have adequate strength to resist tearing or collapsing into chamber 12. Accordingly, it is preferably to include porous support disc 24b between membrane 24a and chamber 12. It may be separate from the membrane or bonded to it, if desired. Support disc 24b may be formed of a flexible plastic material such as polyester or fiberglass, such as supplied by Glassrock Co., and is preferably more rigid than membrane 24a. It is preferably thicker than membrane disc 24a, typically ranging from 1mm to 2mm. It is sufficiently porous so that it does not create undue pressure drops when the liquid sample moves into and out of chamber 12.

Support disc 24b may carry a dry reagent which is soluble in the liquid sample. When the liquid sample flows through disc 24b, it dissolves the reagent for flow to membrane 24a for reaction with the sample component. This saves the step of independently adding a further component. For example, in a sandwich assay for an antigen, one antibody is immobilized on membrane 24a and a soluble labeled antibody is deposited in dry form on support disc 24b, as by lyophilization directly on the support disc. Passage of liquid sample through the support disc carries with it the soluble label antibody which forms a sandwich with the corresponding sample as it is present. If desired, the soluble dry reagent may be deposited on some other portion of the assay device exposed to chamber 12 and so which contacts liquid sample.

In operation, the assay device of the present system broadly is used to determine a predetermined component of a liquid sample with at least one reagent to form a detectable product on membrane disc 24a as an indication that the component is present in the sample. The liquid sample is contacted on one side of membrane means 24 and the chamber volume is varied to selectively cycle the liquid sample through the membrane means into and out of the chamber. To permit complete cycling, the chamber is substantially free of absorbent material.

The system is particularly applicable to an immunoassay wherein the sample component is one component of an immunological pair including antigens, antibodies, or haptens.

The immunological pair includes two components which immunologically bond to each other. Specific immunological pairs include antigens and their antibodies (monoclonal antibodies or polyclonal antibodies), biologically functional haptens and their antibodies, enzymes and their substrates, hormones and their receptors, vitamins and their receptors, biotin and either avidin or an antibody to biotin, and lectin and its specific binding mono,di- or trisaccharide.

For simplicity of description, the system will be described with respect to immunoassays using the antigen-antibody immunological pair. The liquid sample is biologically derived, (e.g., urine or serum) and the one reagent comprises a labeled antigen or antibody.

Referring first to a sandwich assay for the detection of antigen, the one reagent is a labeled antibody specific for the predetermined antigen in the sample. This system uses a second reagent, a capture antibody also specific for the predetermined antigen, immobilized on membrane disc 24a prior to addition of the liquid sample to the device. The techniques of immobilizing proteins such as monoclonal or polyclonal antibodies to solid surfaces such as membrane disc 24a without deactivation are well known. See e.g., Schuurs U.S. 3,551,555 and Hendry et al., J. Immun. Methods, 35 1980, 285. For plastic materials such as nylon, such proteins may be immobilized by covalent bonding, e.g., as described in U.S. 3,720,760. The amount of protein immobilized per unit area of nylon is greater than that for nitrocellulose.

The labeled antibody or antigen described with respect to the sandwich assay may be any of the conventional types including radioactive, enzyme, or a metal complex label which are conjugated to the antibody. Formation of conjugates between such immunological substances and labels are well known, e.g., (a) radioactive labels - U.S. 3,646,346, Hunter et al., Nature 142 (1962), 945, (b) enzyme labels - U.S. 3,654,090, 3,791,931 and 3,817,838, Wilson et al., Immunoflourescense and Related Staining Techniques, Knapp., W. et al., Eds. L. Sevier-North Holland, Bio-Medical Press, New York-Amsterdam, 1978, pp. 215-224; (c) fluorescent quencher labels - U.S. 3,996,345; (d) radioactive labels - U.S. 4,062,733; (e) fluorescent or enzyme labels U.S. 4,067,959; (f) chemiluminescent labels - U.S. 4,104,029; (g) non-enzymatic catalyst label - U.S. 4,160,645; (h) enzyme pair labels - U.S. 4,233,402, chemically induced fluorescent labels - 4,720,450; and (i) enzyme non-ionic charge labels - U.S. 4,287,300. In addition, the labels disclosed in U.S. 4,366,241 may be employed. Also, colloidal gold labels are discussed in detail hereinafter.

When an enzyme is used as a label, the presence of the enzyme label in the reaction product on the surface of the membrane is detected by contact of the enzyme with a substrate in solution which produces a color upon reaction with the enzyme. In the present procedure, after the predetermined number of cycles of the liquid sample, the liquid sample may be discarded and the substrate contacted thereafter with the reaction product on the membrane. As with the liquid sample, the substrate solution may be deposited onto the external surface of the membrane and passed through it by suction created by increasing the volume of chamber 12. Alternatively, it may be initially placed in the chamber and forced out of it by decreasing the chamber volume. Then, the color formed by the substrate is visible on the membrane from its externally exposed side.

Colloidal gold conjugates useful for probes such as cytochemical markers are well known for microscopy. See, e.g., Scanning Electron Microscopy, 1981, II, pp. 9-31, "Immunocytochemistry" Eds. Polak, J.N., et al., Bristol, London, Boston (1982) pp. 82-112, and Journal of Neuroscience Methods, 7(1983), pp.1-18. Colloidal gold particle markers are simple to use in comparison to other conventional markers. For example, they do not require instruments necessary for detection of other markers such as radioactive isotopes. Furthermore, unlike enzymes, they do not require the additional step of adding substrate. However, they have not been used extensively for commercial immunoassay kits, perhaps because of their low level of visibility using conventional techniques for mixing reactants. For example, the sensitivity of an assay using colloidal gold conjugates in a once-through membrane system may be insufficient to provide the desired level of sensitivity. It has been found that by cycling of liquid sample and reagents through the membrane, the mixing is so improved that colloidal gold particle conjugates are useful reagents for immunoassay kits without the need to use expensive microscopes. In that regard, cycling of the liquid sample through the membrane at least 3 times increases the sensitivity of the assay approximately 10-fold or more, while cycling at least 15 times increases the sensitivity approximately 50 to 100-fold or more.

If the sensitivity of the gold-immunological reagent conjugate is insufficient, even with the increased mixing capability of the present system, a technique for enhancing the sensitivity of the gold complex may be employed such as disclosed in Holgate, C.S., et al., J. Histochem. Cytochem 31:938 (1938) and in Dancher, G, et al., J. Histochem. Cytochem 31:1394 (1983). This system is an "indirect" technique employing an immunological reagent, immunoglobulin, absorbed to colloidal gold. The gold particles are revealed by a silver precipitation reaction. In essence, the silver enhancement takes advantage of the catalytic effect of gold to catalyze the photographic physical developer process converting silver ion to silver metal. Suitable colloidal gold or gold sol particle size is from 3nm to 150nm. This immuno gold-silver staining method may have an enhanced sensitivity of up to 200-fold in comparison to the use of the gold particles without silver staining.

The increased sensitivity permitted by cycling is clear from a theoretical model of the immobilized immunological reagent contact with the sample component. The immunological reagent, in this instance, antibody, is immobilized on the membrane either by absorption or covalent bonding. The antibody is exposed to the channels or pores of the membrane and is only capable of capturing antigen in passing sample which is proximate to the antibody. Cycling the sample to permit contact of different sample portions flowing through the membrane pores provides substantially increased chances for reaction between the antigen and the immobilized antibody, thereby not only increasing the sensitivity of the assay but also its speed.

The mixing effect of cycling is particularly apparent when the immunological reagent is concentrated at a specific region, such as a dot on the membrane, as described below. In that instance, the immobilized antibody is only deposited at that one dot. In once-through techniques, only the antigen in the sample in an imaginary column above the dot contacts the antibody. The remaining antigen in the sample passes through the membrane without such contact. In contrast, there is continuous mixing of different sample portions cycling antigen which causes exposure of significantly more antigen to the immobilized antibody on the membrane with the above advantageous results. This principle of increased mixing efficiency applies to any of the conventional immunoassays with respect to which the present system may be employed. Also, the increased efficiency or interaction increase the local concentration of reactants which, in turn, increases the reaction kinetics.

The labeled antibody may be added simultaneously with the sample or may be added before or after the addition of sample. In a particularly preferred embodiment, the labeled antibody is deposited in dry form retained on the porous support disc 24b prior to addition of the liquid sample. Then, when the sample flows in either direction through membrane 24a, it dissolves the labeled antibody so that it reacts with antigen if present in the sample to form a sandwich of immobilized antibody.antigen.labeled antibody on the surface of membrane 24a. The system is also applicable to the detection of antibody by reversal of the antigen and antibody to form a sandwich of immobilized antigen.antibody.labeled antigen (or anti-antibody).

As mentioned above, a step in the analysis is eliminated by the predeposit of labeled antibody on the support disc. This is possible because the liquid sample is cycled backward and forward through the membrane. By forming support disc 24b of an opaque material, detectable label in the liquid sample retained in chamber 12 is not visible on the external exposed surface of membrane 24a and so it does not interfere with signal produced on the upper surface. In effect, support disc 24b also serves as a visual mask between the background signal of unreacted labeled reagent in the chamber and the labeled reaction product on the membrane.

The present device is also applicable to the competitive binding technique. In such system for the detection of antigen in a liquid sample, the corresponding member of the immunological pair, i.e., antibody is immobilized on to the membrane surface. Antigen labeled in the manner described above of the same immunological character as the antigen to be detected in the sample is contacted with the immobilized antibody on the membrane. The immobilized antibody is in limited supply, and so a competition is set up between the antigen in the sample and the labeled antibody. Thus, the signal emitted from the label is inversely proportional to the amount of antigen in the sample. Such competitive binding technique using a membrane is described in Bagshawe U.S. 3,888,629.

As with the sandwich assay, the competitive binding assay may be performed by reversing the roles of the antigen and antibody. In this instance, the immobilized member of the immunological pair is the antigen for the detection of antibody in the sample which competes with labeled antibody.

The competitive binding assay, like the sandwich assay, can be simplified by using the present system. That is, the labeled immunological reagent conjugate, e.g., antigen-label, can be coated onto porous support 24b in lyophilized form. When the liquid sample suspected of containing antigen contacts the support, it dissolves the labeled antigen and carries it to membrane 24 at which the competition between the sample antigen and labeled antigen is set up for the limited supply of antibody at the membrane surface.

The immunoassays which have been described are the sandwich assay and the competitive binding assay. It should be understood that the system is also useful for other immunoassays such as, for example, described in U.S. 4,366,241.

The substances to be analyzed include a wide variety of biologically derived substances, e.g., proteins. The following is a list of some of these substances. (The listed substances also include immunologically reactive antibodies, and fractions of the substances.

Immunoglobulins

    IgE
    IgA
    IgM
    IgD

Microorganisms

Aerobacter aerogenes
Aerobic Spore-forming Bacilli
    Bacillus anthracis
    Bacillus subtilis
    Bacillus cereus
Anaerobic Spore-forming Bacilli
    Clostridium botulinum
    Clostridium tetani
    Clostridium perfringens
Brucellae
    Brucella melitensis
    Brucella abortus
    Brucella suis
Chlamydia (unclassifiable parasites bacterial/viral)
    Chlamydia agents (naming uncertain))
    Chlamydia trachomatis
Corynebacteria
    Corynebacterium diptheriae
Escherichia coli
Fungi
    Cryptococcus neoformans
    Histoplasma capsulatum
    Coccidioides immitis
    Candida albicans
    Mucor corymbifer (absidia corymbifera)
Hemophilus-Pordetella group
    Hemophilus influenzae
    H. ducreyi
    H. hemophilus
    H. aegypticus
    H. parainfluenzae
Keibsiella pneumoniae
Mycobacteria
    Mycobacterium tuberculosis hominis
    Mycobacterium bovis
    Mycobacterium avium
    Mycobacterium leprae
    Mycobacterium paratuberculosis
Mycoplasmas
    Mycoplasma pneumoniae
    Mycoplasma hominis
Neisseriae
    Neisseria meningitidis
    Neisseria gonorrheae
Other Pathogens

Listeria monocytogens
Pasteurellae
Pasteurella pestis
Pasteurella multocida
Pneumococci
Diplococcus pneumoniae
Pseudomonas aeruginosa
Rickettsiae (bacteria-like parasites)
Rickettsia prowazekii
Rickettsia mooseri
Rickettsia rickettsii
Rickettsia conori
Rickettsia australis
Rickettsia tsutsugamushi
Rickettsia burnetii
Salmonella choleraesus
Salmonella typhimurtum
Salmonella typhosa
Shigella arabinotardo
Shigella boydii
Shigella dysenteriae
Shigella flexneri
Shigella schmitzii
Shigella Sonnei
Staphylococci
Staphylococcus aureus
Staphylococcus albus
Streptococci
Streptococcus pyogenes
Groups B, C, D, F, G
The Spirochetes
Treponema pallidum
Borrelia recurrentis
Leptospira icterohemorrhagiae
Leptospira canicola
Toxoplasma gondii

Peptide and Protein Hormones

Corticotropin (ACTH)
(adrenocorticotropic hormone)
Follicle-stimulating hormone
Luteinizing hormone
(interstitial cell-stimulating hormone)
Parathyroid hormone
Prolactin
Chorionic Gonadotropin
Insulin
Glucagon
Relaxin
Somatropin
Triiodothyronine
Thyrocalcitonin
Thyroxine

Tissue Hormones

Angiotensin I and II

8

Bradykinin

Gastrin

Human placental lactogen

Secretin

Peptide Hormones

Oxytocin

Vasopressin Viruses

Adinoviruses

Arboviruses

    Eastern Equine Eucephalitis Virus

    Western Equine Eucephalitis Virus

    Sindbis Virus

    Semliki Forest Virus

    St. Louis Encephalitis Virus

    California Encephalitis Virus

    Colorado Tick Fever Virus

    Yellow Fever Virus

    Dengue Virus

Hepatitis

    Hepatitis A Virus

    Hepatitis B Virus

Herpes Viruses

    Herpes simplex, Types I and II

    Varicella (Chicken pox)

    Cytomegalovirus

Myxoviruses

    Influenza (A, B, and C)

    Parainfluenza (1-4)

    Mumps virus

    Newcastle Disease Virus

    Measles Virus

    Canine Distemper Virus

    Respiratory Syncytial Virus

    Rubella Virus

Picornaviruses

    Poliovirus

    Coxsackievirus

    Echoviruses

    Rhinoviruses

Pox Viruses

    Vaccinia

    Molluscum contagiosum

The system is also applicable to other assay systems which are not categorized as immunoassays, e.g., the detection of unknown DNA sequences. For example, liquid sample containing the unknown DNA sequence is passed through the membrane and immobilize on the membrane as by contact with DNA previously immobilized on the membrane. Then, a labeled DNA probe passed in a liquid through the membrane. If hybridization occurs, the labeled DNA probe will be retained in detectable form on the membrane surface. This system is described in Polsky-Cynkin, R., et al., Clin. Chem. 31/9, 1438 (1985).

As mentioned above, in a particularly effective assay, the immunological reagent is concentrated on at least one defined region on the membrane which appears as a dot but which actually extends through the membrane in a column of a diameter approximately equal to the dot. Referring to a sandwich or competitive binding techniques, this is accomplished by immobilizing the capture immunological reagent, e.g., antigen or antibody, only in such region by flowing the reagent to be immobilized through the membrane thickness. The reaction with the sample component and with the labeled reagent only occurs in that region.

The dot approach has certain advantages such as the performance of multiple simultaneous assays with a single devise as described below. Also it provides a more distinctive end product signal since it is concentrated at a single region. The ability to cycle the sample through the membrane greatly increases the mixing efficiency of the sample and immobilized immunological reagent. In contrast, a conventional once-through system, only the immunological component in the sample exposed to the dot is the imaginary liquid column directly above the dot. Cycling through the membrane permits the immunological component in the remainder of the liquid sample to contact the immobilized reagent to concentrate the sample and labeled reagent. This can permit the detection of a label such as colloidal gold without instrumentation where it would not otherwise be detectable.

Another advantage of the dot approach is that it permits the simultaneous detection of multiple components in a sample. For example, with a single assay device, two different antibodies specific for predetermined different antigens can be immobilized on distinct spots on the membrane. The sample suspected of containing either one of the antigens is then contacted with the membrane and with labeled antibody specific for the two different antigens. A signal produced by the labels at one or the other of the dots indicates the presence or absence of one or both of the antigens. The dots may be distinguished from each other by their location or by an identification near each immobilized antibody dot. Thus, for example, if a color appears at the first dot but not the second, the first antigen but not the second antigen is present. If both dots appear, then both antigens are present, and if no dots appear, neither antigen is present. Alternatively, the label may be selected to produce different colors at each of the dots.

This system could be expanded to include the simultaneous detection of more than two components of the liquid sample by a corresponding number of immobilized immunological reagent on the membrane. In some instances, a first antibody is reactive with a particular subunit of a number of different antibodies. If a second antibody is specific for a subunit of one antigen only, such second antibody can be used as the immobilized antibody and a single labeled first antibody can be used as the universal labeled antibody for antigen of interest.

The standard protocols for the conventional immunoassays may be used with the present device. For example, in a sandwich assay, the order of addition of the sample and labeled reagents may be simultaneous or sequential.

While monoclonal antibodies have known advantages and purity over polyclonal antibodies, either type of immunological reagent can be used in accordance with the present invention.

In operation of the above device, the flow rate of liquid through the membrane is controlled by the pressure in chamber 12. Thus, if the liquid is on the exterior surface of the membrane, the rate of increase in the volume of the chamber 12 causes a corresponding increase in the flow rate through the membrane. Conversely, if the liquid is in the chamber, the liquid flow rate through the membrane is controlled by the rate of decreasing the volume of the chamber. Typical flow rates through the membrane using the present device are on the order of 0.5 ml/second depending on the porosity of the membrane and viscosity of the fluid.

Modification of the specific device illustrated may be made so long as a membrane is substantially sealed across an opening into a variable volume chamber and means is provided for readily varying the volume of such chamber. For example, if desired, the piston could be a slideably received plunger within the body to vary the volume of the chamber with a non-twisting push and pull force applied to the piston or body. In this device or the one illustrated in the drawings, a stop may be included to prevent disengagement of the body and piston during normal cycling of the liquid. On the other hand, there are certain advantages in permitting the ready disengagement of the piston and body so that the liquid in the chamber can be readily emptied without passage through the membrane or such liquid may be placed in the chamber in the first instance. Also, other more elaborate means may be provided for varying the volume of the chamber.

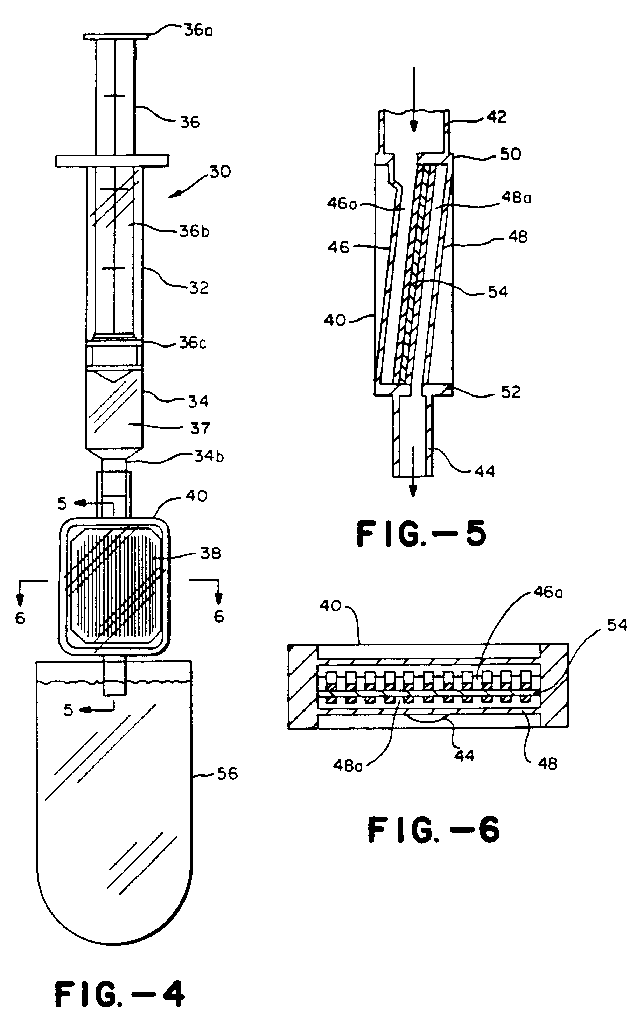

Referring to Figs. 4, 5 and 6, a slideable plunger or piston assay device 30 is illustrated. In this instance, the slideable piston is used instead of the screw threaded piston of the device of Figs.. 1-3. The device includes three separate functional portions which are mated together during operation.

One portion comprises piston means 32 including a piston housing 34 within which a piston plunger 36 is slideably received. Housing 34 includes a tip 34b. Plunger 36 includes a handle portion 36a at one end, a piston rod 36b and a slideable piston head 36c with a flexible rubber perimeter which forms a slideable seal with the inner wall of the cylindrical housing 34. As illustrated, the piston means 32 is in the form of a hypodermic syringe. The interior wall of the housing 34 and the forward end of the piston head 36c define a chamber 37 of variable volume sealed from the exterior except through an outlet port in the tip 34b.

A membrane means 38 is in a self-contained housing 40 formed independently of the piston means 32. One advantage of this configuration is that a conventional hypodermic syringe can be used in combination with the membrane means by a simple force fit. Alternatively, the two parts can be integrally formed.

The housing 40 includes an inlet port 42 and an outlet port 44. The housing 40 is formed of support walls 46 and 48 integrally connected to transverse walls 50 and 52 through which project the inlet and outlet ports 42 and 44, respectively. Sandwiched between the flat walls 46 and 48 is a membrane 54. One, and preferably both, of the walls 46 and 48 are either totally transparent or include a transparent window so that the membrane 54 can be viewed from the exterior of the device. The walls 46 and 48 are formed with adjacent stepped ridges and grooves defining parallel channels 46a and 48a, respectively, aligned with the direction of flow of fluid entering the port 42 from the syringe. The channels 46a are closed at one end by end wall 52 and the channels 48a are closed at one end by end wall 50 so that the only path of fluid entering the inlet port 42 is through the membrane 54 and out outlet port 44.

The liquid leaving the port 44 can be collected in a suitable receptacle such as a test tube 56. Alternatively, not shown, the outlet port 44 may mate with a port of an enclosed vessel for ready withdrawal of the fluid on the application of suction force by piston means 32, as described below.

The function of the assay device of Figs. 4-6 is similar to that of the device of Figs. 1-3. Membrane 54 corresponds to membrane 24a. The same type of reactions set forth above may be performed on membrane 54, including the use of the dot technique. Since walls 46 and/or 48 are transparent, the reaction product, including a detectable label, is visible to the eye through the transparent wall.

In one mode of operation, the liquid initially is deposited in receptacle 56 and tip 34b is immersed. Plunger 36 is fully inserted in housing 34 so that chamber 37 is at minimum volume. Then, plunger 36 is withdrawn to increase the volume of chamber 37 and create suction which draws fluid from receptacle 56 through port 44 and channels 48a to pass across membrane 54 into channels 46a and out port 42 into the chamber 37.

Alternatively, the liquid sample may be initially in place in chamber 37 and forced under pressure through the port in the tip 34b into port 42 and channels 46a across membrane 54 and channels 48a into outlet port 44 into receptacle 56. As with the device of Figs. 1-3, the fluid may be cycled back and forth through membrane 54 to increase the mixing efficiency.

The use of a self-contained membrane housing in conjunction with a syringe has been known in the art of filtration. A self-contained membrane housing similar to the type illustrated in Figs. 4-6 is sold by Costar of Cambridge, Massachusetts. However, there is no suggestion for using such a filtration device in the assay system of the present invention or of cycling through the membrane.

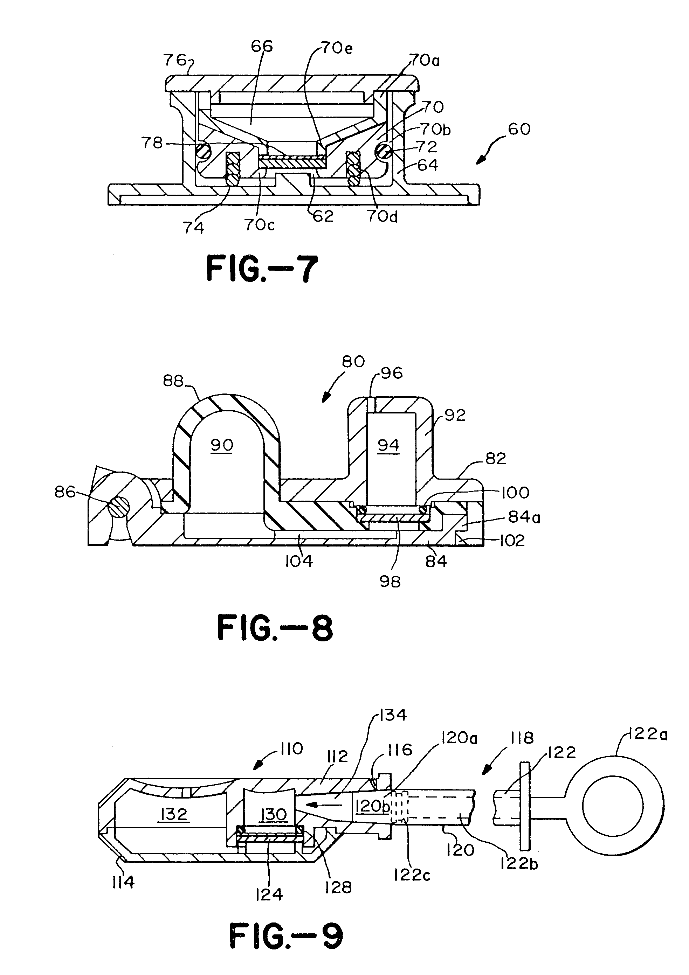

In Fig. 7 the device of Fig. 1 is modified so that the piston member is mounted for slideable rather than twisting movement. Specifically, the assay device is illustrated in the form of container means 60 defining a hollow interior space or chamber 62 of variable volume. Container means 60 includes a hollow body 64 and piston means 66 mounted for movement into and out of the body upon relative sliding movement between the piston means and body. Similar to the device of Figure 1, body 64 is a generally U-shaped vertical cross-section and a circular horizontal cross-section. Body 64 may be suitably formed of the same materials as body 14 of Fig. 1.

Piston means 66 includes a circular piston member 70 with an exterior upright wall portion 70a and an annular recess 70b for O-ring 72 which serves to maintain a pressure seal against the inner wall of body 64 when piston member 70 is moved. Piston member 70 also includes an inner wall 70c which with horizontal bottom portion of body 64, defines chamber 62. Piston member 70 also includes another annular opening 70d within which is fitted spring means in the form of coil spring 74 which is fully compressed in Fig. 7. When the pressure is released, spring 74 will move piston member 70 upward and out of body 64 to expand chamber 62.

An optional top 76 is provided with a downwardly projecting rim to be seated against the corresponding shoulder of body 64 to facilitate pressing piston member 70 toward body 64.

Membrane means 78 is retained in the open center of piston 66 by pressure fit against lower projecting portion of piston wall segment 70e. As illustrated, membrane means 78 includes a circular porous membrane in the form of a membrane disk with an externally visible upper surface and independent support below it. Membrane 78 may be unitary or segmented as illustrated and may take the form,described with respect to membrane 24 above.

The device of Fig. 7 is illustrated with chamber 62 at the minimum volume. When the pressure on spring 74 is released, the spring will cause the volume of shoulder 62 to expand as desired. The system may be operated substantially as described with respect to the device of Fig. 1.

Referring to Fig. 8, a push-button device 80 is illustrated which also functions on the variable volume principle described above. Device 80 includes an upper housing 82 which pivots from a lower housing 84 about a rod 86 which serves as a pivot point. Lower housing 84 is permanently fixed to rod 86 as by a force fit while housing 82 is free to rotate between a horizontal and a vertical position, if desired, to permit the flow of liquid into the device.

Upper housing 82 includes a push-button or bulb 88 formed of a resilient, collapsible material such as rubber, mounted to the remainder of upper housing 82 by welding or the like. Bulb 88 defines a variable volume chamber 90. Bulb 88 is suitably sealed with the remainder of upper housing 82 by welding or the like. Upper housing 82 also includes a dome portion 92 which may be rigid and which defines a fixed volume chamber 94 and which includes an opening 96 to release excess pressure. Below chamber 94 is mounted membrane means 98 suitably retained in place by a gasket 100. Since the reaction product is viewed on membrane 98 through the top of dome 92, it should be transparent, or at least translucent. Upper housing 82 also includes a flexible L-shaped locking portion 102 which fits into a corresponding rim 84a of lower housing 84 to retain the unit in a closed position until it is desired to reopen it. The system can be readily unsealed by pivoting locking portion 102 away from shoulder 84a and then pivoting upper housing 82.

Interconnecting chamber 90 and 94 is a flow channel or bore 104 formed between the upper and lower housings in the form of a circular bore or the like communicated between chamber 90 and the lower portion of the membrane means 98.

In operation, either chamber 90 or 94 may be partially filled with liquid prior to closing the system. When chamber 90 is filled and the liquid is to be moved from chamber 90 through membrane means 98 and to chamber 94, bulb 88 is pushed downwardly to reduce the volume of chamber 90 and to cause the liquid to flow through membrane means 98 into chamber 94. When it is desirable to move the liquid in the opposite direction, the pressure on bulb 88 is released and the solution is drawn under the thus-created reduced pressure back into chamber 90. If the liquid initially is in chamber 90, it is pushed in the first pass through membrane 98 under positive pressure rather than vacuum and is only drawn back into chamber 90 by reduced pressure caused by release of the bulb. Similar assays may be performed as described regarding the device of Fig. 8.

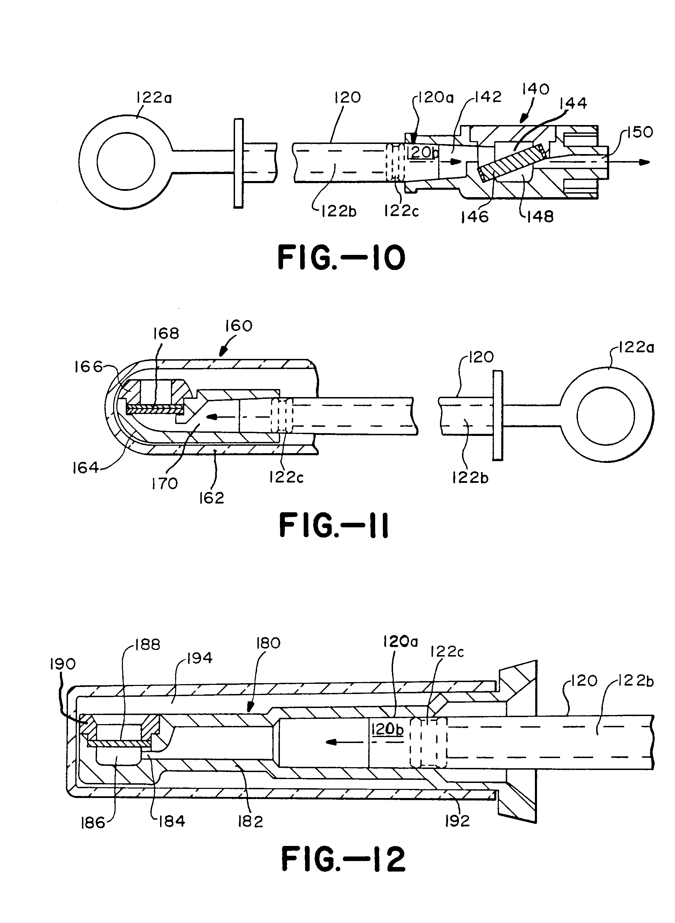

The device of Fig. 9 is similar to that of Fig. 4 in that the driving force may be provided by a conventional or modified syringe which may be integral or removable from the device of figure 4. The device 110 includes an upper housing portion 112 and a lower housing portion 114 which are retained as an integral piece by fusion at the interfaces or the like. The device defines an open end 116 where it is in sealing engagement with a syringe 118. The syringe includes a piston housing 120 within which a piston plunger 122 is slideably received. As illustrated, piston plunger 122 includes a ring 122a for convenient holding of the device in one hand and movement of the piston into and out of piston housing 120. Also included are a piston rod 122 b and the slideable piston head 122c. Piston body 120 includes a tip 120a which forms a removable seal with the wall defining opening 116.

Membrane means 124 is mounted between upper and lower housings 112 and 114, respectively, in cooperation with gasket 128. Rigid chambers 130 and 132 communicate with each other on opposite sides of membrane means 124.

In operation of device 110, a solution initially within chamber 120b defined to the forward side of piston head 122c is forced under pressure through channel 134 into chamber 130 downwardly and through membrane means 124 and into chamber 132. To move the solution in the opposition direction, piston head 122c is pulled in the opposition direction by ring 122a to create a reduced pressure in chamber 120b to cause the liquid to be drawn back and upwardly through the membrane through chamber 130. The reaction product is viewed on the upper surface of membrane means 124 and so the portion of upper housing 112 above the member is transparent.

Referring to the device of Fig. 10, another integral housing is illustrated like that of Fig. 9 for use with an external syringe, such as the one illustrated in Fig. 9. Accordingly, like numbers will be used for the syringe and the device of Fig. 10 as well as that of Figs. 11 through 13 to be described. Device 140 includes an opening 142 which seals against tip 120a of the syringe and communicates with a chamber 144 across membrane 146 into lower chamber 148 and through open channel 150. In operation, channel 150 may be placed into the test tube from which a solution may be drawn by pulling ring 122a to the left and creating reduced pressure in chamber 120b. This causes the liquid to flow through channel 150, chamber 148, membrane means 146, chamber 144, opening 142 and into chamber 120b. The portion of the housing above chamber 144 is transparent for viewing. By moving the piston rod 122b in the opposite direction, the liquid flows in the reverse direction.

The device 160 of Fig. 11 is also operable with a removeable syringe. As illustrated, it is disposed within a test tube 162 from which it may draw solution as described. Device 160 includes a lower body 164, an upper body 166, and membrane means 168 which is retained therebetween. Body 166 defines a channel 170 between tip 120a and the lower side of membrane means 168. The lower body 164 is generally tubular in external shape while upper body 166 is generally of circular plug shape with an open central circular area through which the upper portion of the membrane may be viewed.

In operation of the device, the liquid initially is within the syringe or within test tube 162 and may be

EP 0 246 760 B1

forced to flow in either direction through membrane means 168 as described above.

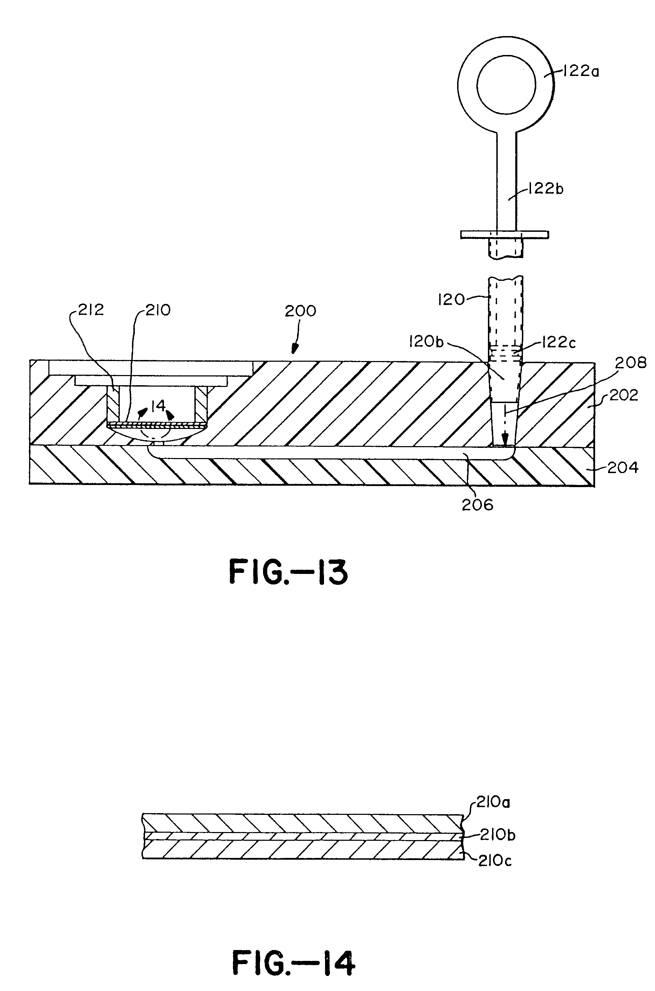

Referring to Fig. 12, a modification of the device of Fig. 11 is illustrated with a seal formed with an external container for the device.

The device 180 includes a hollow tubular member 182 with stepped inner dimensions. A slight taper is provided so that the tip 120a is pressure fit to form a seal within one segment of the device. Tubular member 182 defines a narrow channel 184 into a lower chamber 186 below membrane means 188. An upper circular piece 190 of similar construction to piece 166 in Fig. 11 is provided to retain membrane means 188 in a seated sealed position. The exterior of tubular member 182 includes a stepped outer diameter which is forcefit for removeable sealing engagement in an exterior transparent tube 192.

In operation of the device of Fig. 12, if liquid is initially within chamber 12b, it is forced under pressure from the syringe through channel 184 into chamber 186 and through membrane means 188 upwardly into the well formed by piece 190, and, if sufficient liquid is present, into the annular space 194 defined between tube 192 and member 182. When piston 122 is withdrawn, the liquid can flow in the opposite direction through membrane means 188.

The device 200 of Figs. 13 and 14 includes an upper housing portion 202 and a lower housing portion 204 defining therebetween a bore 206. Upper housing portion 202 defines a tapered circular hole 208 within which tip 120 b of the syringe is forcefit for sealing engagement.

Membrane means 210 is held by a force fit by an annular plastic retaining ring 212 into a horizontal position in upper housing portion 202. The upper surface of the membrane means is externally visible since ring 212 define open space to its interior. The membrane means 210 includes three layers, an upper layer 210a which may be formed of paper-backed nitrocellulose, polyester backed nitrocellulose, nylon or modified nylon; a middle layer 210b, a filter, which may be formed of the same materials, and a lower support disc 210c which may be formed of porous plastic, fiberglass or the like. Alternatively, a single layer may be used. Any of the layer may be impregnated with lyophilized reagent such as a colloidal gold conjugate.

In operation, the device of Figs. 13 and 14, operates by forcing liquid from the syringe through the bore 206 upwardly through membrane means 210. The liquid may move in the opposite direction by withdrawing the piston of the syringe.

While the above system is described in terms of yes-no qualitative tests, it should be understood that it is also suitable as a semi-quantitative test using appropriate signals, such as colors, produced at different known concentrations of the component to be analyzed. Thus, for example, for the analysis of an antigen by a sandwich technique, the system can be run at progressive dilution to obtain an approximation of the color expected for a particular dilution. The unknown concentration of antigen is compared to these colors to give an approximation of the concentration of antigen present in the sample.

Methods according to the present invention are provided by the following specific examples. It should be understood that the data disclosed serve only as examples and not intended to limit the scope of the invention.

Example 1

In this example, a sandwich immunoassay is illustrated for the antigen human chorionic gonadotropin (hCG) in a urine sample containing a known amount of hCG. Monoclonal antibodies were used for the immobilized reagent and for the labeled reagent. The labeled conjugate comprises a gold sol based upon the procedure of Faulk, W.P., et al. Immunochem. 8:1081 (1971). Briefly summarized, 100ml distilled water containing 0.01% chloro-auric acid solution is mixed with 4ml of 1% trisodium citrate and boiled for 15 minutes. The solution turns from yellow to red at an optical density at 520nm of about 1.

The other immunological reagent is an anti-alpha chain hCG monoclonal antibody supplied by Medix Biotech, Inc.. A membrane disc 24a (supplied by Schleicher and Scherell, 0.45 microns porosity and 140 microns thick) is used. The anti-alpha monoclonal antibody ($1\mu\ell$) is applied to the disc at a concentration of 1 mg/ml in a limited area of about 203mm diameter. The innoculated disc is then incubated with BSA in phosphate buffered saline solution and placed into the device described above.

Monoclonal antibody to the beta chain of hCG (supplied by Miles Laboratories) is conjugated with this gold sol and is coated onto the porous disc 24b and is frozen in a -45°C (-50°F) temperature bath. Then it is freeze-dried.

A 500 microliter urine sample is placed onto the top of the membrane with chamber 12 of its minimum volume as illustrated in Fig. 3a. Then the piston is turned relative to the body to cause the chamber to expand as illustrated in Fig. 3b. The sample solution is then drawn through both membrane 24a and porous support disc 24b containing the lyophilized labeled antibody. During passage through disc 24b, the labeled

13

antibody is dissolved in the urine. Then, piston member 16 is twisted into the opposite direction to force the urine sample out of the chamber through the membrane. This cycle is repeated a number of times with increasing color during each cycle. In this way, the sensitivity of the assay is increased by cycling the liquid through the membrane. This permits the visual reading of a red dot on the membrane using colloidal gold particles at antigen concentrations where this would not normally be done.

The above procedure is repeated using different membrane porosities, concentration of hCG antigen, and number of passages until a red color was produced by the colloidal gold. The results are set forth in the following table.

### Membrane Porosity: 0.45 microns

| hCG Concentration (mIU/ml) | 3.5 | 10 | 20 | 103 | 206 |
|---|---|---|---|---|---|
| Number of Passages Until Red Color is Observed | 7 | 7 | 5 | 3 | 1 |

### Membrane Porosity: 12 microns

| hCG Concentration (mIU/ml) | 3.5 | 10 | 20 | 103 | 206 |
|---|---|---|---|---|---|
| Number of Passages Until Red Color is Observed | — | 15 | 10 | 7 | 3 |

Example 2

In this instance, a sandwich assay is performed in which antigen is immobilized on the membrane, an antibody component of a serum sample is to be analyzed, and labeled antibody lyophilized on the plastic support disc is a conjugate of an anti-antibody to the sample antibody labeled with colloidal gold formed in the manner described above. In this instance, the antigen, M.pneumoniae is impregnated onto a nitrocellulose membrane disc as described in Example 1. The serum suspected of containing antibodies to the antigen is cycled through the membrane five times by pumping as described above. Then it is washed by phosphate buffered saline solution. Thereafter, the labeled anti-antibody (secondary antibody) is passed through the membrane several times by pumping. In this instance, the secondary antibody is linked to 15nm colloidal gold ($OD_{520}$ = 3.0). A positive reaction is indicated by a distinct red dot on the membrane.

Example 3

In this Example, the procedure of Example 2 is followed, except that the membrane disc includes two different dots, one containing M.pneumoniae antigen and the other Adenovirus antigen. If the serum sample contains antibody to M.pneumoniae alone, a single dot in a pre-selected position is obtained similarly. If the serum contains antibodies to adenovirus alone, a single dot at another predetermined position is indicated. If it contains antibodies to both M.pneumoniae and adenovirus two dots will appear. If it contains antibodies to neither, no dots will appear.

Example 4

In this instance, a competitive binding assay is performed for thyroxine antigen. The thyroxine, a hapten, is formed into a colloidal gold conjugate for use in competition with the sample suspected of containing thyroxine. The conjugate is formed by reacting the thyroxine with colloidal gold conjugate through spacer arms formed of horseradish peroxidase. A saturated solution of thyroxine sodium salt in 0.1M sodium bicarbonate solution (10ml) at pH 9.5 is conjugated through horseradish peroxidase using the procedure of Nakane, P.K., J. Histochem Cytochem, 14:929 (1966). In this instance, the thyroxine solution is used instead of the protein described in Nakane.

After conjugation, the solution is dialyzed against distilled water to remove excess thyroxine. The thyroxine-horseradish peroxidase conjugates are linked to 10nm colloidal gold particles and then centrifuged to remove excess non-conjugated free thyroxine-horseradish peroxidase. The residue is resuspended in buffer.

The anti-thyroxine antibody is immobilized on the membrane as follows: Affinity purified rabbit anti-thyroxine antibodies in pH 7.4, 0.01M phosphate buffered saline coated onto a spot on the nitrocellulose membrane is described in Example 1. The concentration of antibody is 100 $\mu$g/ml.

In the testing procedure, 0.1 $\mu\ell$ of the labeled thyroxine and sample containing different known concentrations (1, 5, 10 and 20ng/ml) of thyroxine is applied to the top surface of the membrane 24a of Fig. 1. Different colors are produced at the different concentrations of thyroxine, a more intense color corresponding to a lower concentration of thyroxine. Then, an unknown sample is analyzed and compared against the colors produced by the known concentrations described above.

## Claims

1. An assay device (10; 30; 80; 110; 140; 160; 180; 200) for the detection of one or more predetermined components of a liquid sample Comprising a container (14; 34; 60; 112; 120; 164; 182; 202, 204) defining a chamber (12; 37; 62; 90; 120b, 130; 120b, 144; 170; 120b, 184, 186; 120b, 208, 206) of variable volume, means (16; 36; 66; 88; 118) for varying the volume of the chamber, an inlet port in fluid communication with the chamber, and a means (24; 54; 78; 98; 124; 146; 168; 188; 210) is disposed across the fluid path through the inlet port, characterised in that the said means disposed across the fluid path comprises a fluid permeable membrane means, a reagent reactive with the sample component in a complementary binding assay is retained within the membrane means, and the chamber (12; 37; 62; 90; 120b, 130; 120b, 144; 170; 120b, 184, 186; 120b, 208, 206) is essentially fluid sealed from the exterior of the container except through the membrane means (24; 54; 78; 98; 124; 146; 168; 188; 210), whereby a liquid sample on the membrane means outside of the chamber (12; 37; 62; 90; 120b, 130; 120b, 144; 170; 120b, 184, 186; 120b, 208, 206) is drawn through the membrane into the chamber by suction created upon actuation of the volume varying means (16; 36; 66; 88; 118) to increase the volume of the chamber, and the liquid sample within the chamber is forced out of it under internal pressure created upon actuation of the volume varying means (16; 36; 66; 88; 118) to decrease the volume of the chamber.

2. An assay device as claimed in claim 1, in which the volume varying means comprises a movable wall (16) means forming part of the container means or a resilient, collapsible wall (88) defining part of the chamber and accessible from the exterior of the device.

3. An assay device as claimed in claim 1, wherein the chamber (12, 62) is formed by a hollow body (14, 64) and a piston (16, 66) is mounted within the body for movement into and out of the body upon relative movement between the piston and the body.

4. An assay device as claimed in claim 3, in which the piston (16) is threadedly mounted (14b, 18e) for movement into and out of the body (14) upon relative rotation between the piston and body, the hollow body and the piston means preferably having externally accessible gripping surfaces (14a, 18a) by which the body and the piston can be grasped and twisted manually to vary the volume of the chamber, or in which the piston means is mounted for sliding movement into or out of the body.

5. An assay device as claimed in claim 4, in which the piston is detachable from the hollow body.

6. An assay device as claimed in any preceding claim, in which the sample component comprises one member of an immunological pair and the reagent comprises the other member of the immunological pair, the reagent preferably comprising an antigen or an antibody immobilized on the membrane means.

7. An assay device as claimed in any preceding claim, which comprises a rim (18b) around the membrane means to retain the liquid sample.

8. An assay device as claimed in any preceding claim, in which the membrane means (24, 210) comprises a membrane (210a, 210b) and an adjacent porous support disc (24b; 210c), a dry labeled reagent soluble in liquid sample optionally being disposed on the support disc.

**9.** An assay device as claimed in any one of claims 1 to 7, in which a dry labeled reagent soluble in the liquid sample is disposed on a surface in fluid communication with the chamber.

**10.** An assay device as claimed in any preceding claim, in which the chamber is substantially free of absorbent material.

**11.** An assay device as claimed in any preceding claim, in which the membrane means (54, 98; 124; 146) includes a surface outside of the chamber (37, 90) and which is exposed to the environment or is covered by a transparent wall (46, 48,92).

**12.** A method of detecting at least one predetermined component in a liquid sample, comprising
(a) contacting the liquid sample with one side of a fluid permeable membrane means (24; 54; 78; 98; 124; 146; 168; 188; 210) disposed across an opening into a chamber (12; 37; 62; 90; 120b, 130; 120b, 144; 170; 120b, 184, 186; 120b, 208, 206) otherwise substantially sealed from its surroundings, the chamber being of variable volume, and
(b) varying the chamber volume to move the liquid sample through the membrane means (24; 54; 78; 98; 124; 146; 168; 188; 210) into or out of the chamber (12; 37; 62; 90; 120b, 130; 120b, 144; 170; 120b, 184, 186; 120b, 208, 206) and again varying the volume of the chamber to move the liquid sample back through the membrane means, whereby the predetermined component may react with at least one reagent in a complementary binding assay to form a detectable reaction product on the membrane means as an indication that the component is present in the liquid sample.

**13.** A method as claimed in claim 12, in which the predetermined component is an immunological substance, and the reagent comprises a labeled immunological reagent.

**14.** A method as claimed in claim 13, in which the labeled immunological reagent comprises a conjugate of an immunological substance with a gold particle label.

**15.** A method as claimed in claim 13 or claim 14, in which a second reagent is immobilized on the membrane means prior to contact with the liquid sample, the immobilized reagent being immunologically reactive with the predetermined component.

**16.** A method as claimed in claim 15, in which (a) the labeled reagent is immunologically reactive with the predetermined component but not with the immobilized reagent so that a detectable sandwich-type reaction product on the membrane means is formed by the binding of the labeled reagent to the predetermined component which is bound to the second reagent, or (b) the labeled reagent is of the same immunological type as the predetermined component and so is immunologically reactive with the immobilized reagent but not with the labeled reagent so that a competitive binding assay occurs on the membrane means.

**17.** A method as claimed in any one of claims 13 to 16, in which the labeled reagent is deposited in a dry soluble form on a surface of the device exposed to the chamber, and is dissolved in the liquid sample upon its passage through the membrane means.

**18.** A method as claimed in any one of claims 13 to 17, wherein (I) in step (a) the liquid sample is deposited in the chamber (12), and in step (b) it is moved through the membrane means (24) out of the chamber (12) by decreasing the chamber volume, or (II) in step (a) the liquid sample is first deposited on the exterior of the membrane means (24) and in step (b) it is drawn by suction through the membrane means into the chamber (12) by increasing the chamber volume.

**19.** A method as claimed in claim 18 (I), in which the liquid sample is retained at the exterior surface of the membrane means.

**20.** A method as claimed in claim 18 (I) and further comprising the step of
(c) thereafter increasing the chamber volume to draw liquid sample back from the exterior surface of the membrane means (24) into the chamber (12).

**21.** A method as claimed in claim 18 (II) further comprising the step of
(c) thereafter decreasing the chamber volume to force liquid sample back through the membrane means (24) and out of the chamber (12).

**22.** A method as claimed in any one of claims 13 to 21, in which the reagent is concentrated on at least one defined region on the membrane means and the detectable reaction product is formed at the one defined region, and the membrane means optionally includes a second reagent concentrated on at least a second defined region spaced from the first defined region, the second reagent being reactive with a second predetermined component of the liquid sample, if present, to form a second detectable reaction product at the second defined region.

**Revendications**

**1.** Dispositif analytique (10 ; 30 ; 80 ; 110 ; 140 ; 160 ; 180 ; 200) pour la détection d'un ou plusieurs constituants prédéterminés d'un échantillon liquide, comprenant un récipient (14 ; 34 ; 60 ; 112 ; 120 ; 164 ; 182 ; 202, 204) définissant une chambre (12 ; 37 ; 62 ; 90 ; 120b, 130 ; 120b, 144 ; 170 ; 120b, 184, 186 ; 120b, 208, 206) de volume variable, un moyen (16 ; 36 ; 66 ; 88 ; 118) pour faire varier le volume de la chambre, un orifice d'entrée en communication de fluide avec la chambre, et un moyen (24 ; 54 ; 78 ; 98 ; 124 ; 146 ; 168 ; 188 ; 210) qui est disposé en travers du trajet de fluide passant par l'orifice d'admission, caractérisé en ce que le moyen disposé en travers du trajet de fluide comprend un système de membrane perméable aux fluides, un réactif pouvant réagir avec le constituant de l'échantillon dans une analyse par liaison complémentaire est retenu à l'intérieur du système de membrane, et la chambre (12 ; 37 ; 62 ; 90 ; 120b, 130 ; 120b, 144 ; 170 ; 120b, 184, 186 ; 120b, 208, 206) est pratiquement étanche aux fluides depuis l'extérieur du récipient à l'exception du système de membrane (24 ; 54 ; 78 ; 98 ; 124 ; 146 ; 168 ; 188 ; 210), un échantillon liquide sur le système de membrane à l'extérieur de la chambre (12 ; 37 ; 62 ; 90 ; 120b, 130 ; 120b, 144 ; 170 ; 120b, 184 ; 186 ; 120b, 208, 206) étant ainsi entraîné à travers la membrane dans la chambre par l'aspiration créée par actionnement du moyen de variation de volume (16 ; 36 ; 66 ; 88 ; 118) pour accroître le volume de la chambre, et l'échantillon liquide à l'intérieur de la chambre est forcé hors de cette chambre sous la pression intérieure engendrée par actionnement du moyen de variation de volume (16 ; 36 ; 66 ; 88 ; 118) pour réduire le volume de la chambre.

**2.** Dispositif analytique suivant la revendication 1, dans lequel le moyen de variation de volume comprend un système de paroi mobile (16) faisant partie du récipient ou bien une paroi élastique déformable (88) définissant une partie de la chambre et accessible depuis l'extérieur du dispositif.

**3.** Dispositif analytique suivant la revendication 1, dans lequel la chambre (12, 62) est constituée par un corps creux (14, 64) et un piston (16, 66) est monté dans le corps de manière à se déplacer à l'intérieur et hors du corps par un mouvement relatif entre le piston et le corps.

**4.** Dispositif analytique suivant la revendication 3, dans lequel le piston (16) est monté par l'intermédiaire de filets (14b, 18e) de manière à se déplacer à l'intérieur et hors du corps (14) par rotation relative entre le piston et le corps, le corps creux et le piston ayant de préférence des surfaces de préhension (14a, 18a) extérieurement accessibles par lesquelles le corps et le piston peuvent être saisis et soumis manuellement à une torsion pour faire varier le volume de la chambre, ou bien dans lequel le piston est monté de manière à glisser à l'intérieur et hors du corps.

**5.** Dispositif analytique suivant la revendication 4, dans lequel le piston est séparable du corps creux.

**6.** Dispositif analytique suivant l'une quelconque des revendications précédentes, dans lequel le constituant de l'échantillon comprend un membre d'une paire immunologique et le réactif comprend l'autre membre de la paire immunologique, le réactif comprenant de préférence un antigène ou un anticorps immobilisé sur le système de membrane.

**7.** Dispositif analytique suivant l'une quelconque des revendications précédentes, qui comprend un rebord (18b) autour du système de membrane pour retenir l'échantillon liquide.

**8.** Dispositif analytique suivant l'une quelconque des revendications précédentes, dans lequel le système

17

de membrane (24, 110) comprend une membrane (210a, 210b) et un disque poreux adjacent de support (24b ; 210c), un réactif marqué sec soluble dans l'échantillon liquide étant facultativement placé sur le disque de support.

**9.** Dispositif analytique suivant l'une quelconque des revendications 1 à 7, dans lequel un réactif marqué sec soluble dans l'échantillon liquide est placé sur une surface en communication par un fluide avec la chambre.

**10.** Dispositif analytique suivant l'une quelconque des revendications précédentes, dans lequel la chambre est pratiquement dépourvue de matière absorbante.

**11.** Dispositif analytique suivant l'une quelconque des revendications précédentes, dans lequel le système de membrane (54, 98 ; 124 ; 146) comprend une surface à l'extérieur de la chambre (37, 90) et qui est contact avec le milieu ambiant ou est recouverte d'une paroi transparente (46, 48, 92).

**12.** Méthode de détection d'au moins un constituant prédéterminé dans un échantillon liquide, consistant
(a) à mettre en contact l'échantillon liquide avec une face d'un système de membrane perméable aux fluides (24 ; 54 ; 78 ; 98 ; 124 ; 146 ; 168 ; 188 ; 210) placé en travers d'un orifice dans une chambre (12 ; 37 ; 62 ; 90 ; 120b, 130 ; 120b, 144 ; 170 ; 120b, 184, 186 ; 120b, 208, 206) par ailleurs pratiquement isolée de manière étanche de son environnement, la chambre ayant un volume variable, et
(b) à faire varier le volume de la chambre pour faire passer l'échantillon liquide à travers le système de membrane (24 ; 54 ; 78 ; 98 ; 124 ; 146 ; 168 ; 188 ; 210) à l'intérieur ou hors de la chambre (12 ; 37 ; 62 ; 90 ; 120b, 130 ; 120b, 144 ; 170 ; 120b, 184, 186 ; 120b, 208, 206) et à faire de nouveau varier le volume de la chambre pour refaire passer l'échantillon liquide à travers le système de membrane, le constituant prédéterminé pouvant ainsi réagir avec au moins un réactif dans une analyse de liaison complémentaire pour former un produit détectable de réaction sur le système de membrane comme indication de la présence du constituant dans l'échantillon liquide.

**13.** Méthode suivant la revendication 12, dans laquelle le constituant prédéterminé est une substance immunologique et le réactif comprend un réactif immunologique marqué.

**14.** Méthode suivant la revendication 13, dans laquelle le réactif immunologique marqué comprend un conjugué d'une substance immunologique avec un marqueur constitué d'une particule d'or.

**15.** Méthode suivant la revendication 13 ou la revendication 14, dans laquelle un second réactif est immobilisé sur le système de membrane avant le contact avec l'échantillon liquide, le réactif immobilisé étant immunologiquement réactif avec le constituant prédéterminé.

**16.** Méthode suivant la revendication 15, dans laquelle (a) le réactif marqué est immunologiquement réactif avec le constituant predéterminé mais non avec le réactif immobilisé, de sorte qu'un produit détectable de réaction du type sandwich sur le système de membrane soit formé par la liaison du réactif marqué au constituant prédéterminé qui est lié au second réactif, ou (b) le réactif marqué est du même type immunologique que le constituant prédéterminé et, ainsi, est immunologiquement réactif avec le réactif immobilisé mais non avec le réactif marqué, de sorte qu'un essai de liaison compétitive se produise sur le système de membrane.

**17.** Méthode suivant l'une quelconque des revendications 13 à 16, dans laquelle le réactif marqué est déposé sous une forme soluble sèche sur une surface du dispositif en contact avec la chambre, et est dissous dans l'échantillon liquide lors de son passage à travers le système de membrane.

**18.** Méthode suivant l'une quelconque des revendications 13 à 17, dans laquelle (I), dans l'étape (a), l'échantillon liquide est déposé dans la chambre (12) et, dans l'étape (b), il est amené à passer à travers le système de membrane (24) hors de la chambre (12) par réduction du volume de la chambre, ou (II), dans l'étape (a), l'échantillon liquide est tout d'abord déposé sur l'extérieur du système de membrane (24) et, dans l'étape (b), il est passé par aspiration à travers le système de membrane dans la chambre (12) par accroissement du volume de la chambre.

**19.** Méthode suivant la revendication 18 (I), dans laquelle l'échantillon liquide est retenu au niveau de la surface extérieure du système de membrane.

**20.** Méthode suivant la revendication 18 (I), comprenant en outre l'étape consistant

(c) à accroître ensuite le volume de la chambre pour refaire passer l'échantillon liquide de la surface extérieure du système de membrane (24) dans la chambre (12).

**21.** Méthode suivant la revendication 18 (II), comprenant en outre l'étape consistant

(c) à réduire ensuite le volume de la chambre pour refaire passer à force l'échantillon liquide à travers le système de membrane (24) et hors de la chambre (12).

**22.** Méthode suivant l'une quelconque des revendications 13 à 21, dans laquelle le réactif est concentré sur au moins une région définie présente sur le système de membrane et le produit détectable de réaction est formé au niveau de cette région définie, et le système de membrane comprend facultativement un second réactif concentré sur au moins une seconde région définie distante de la première région définie, le second réactif pouvant réagir avec un second constituant prédéterminé de l'échantillon liquide, s'il est présent, pour former un second produit détectable de réaction au niveau de la seconde région définie.

**Patentansprüche**

**1.** Testvorrichtung (10; 30; 80; 110; 140; 160; 180; 200) für die Feststellung von einem oder mehreren vorbestimmten Bestandteilen einer Flüssigkeitsprobe
- mit einem Behälter (14; 34; 60; 112; 120; 164; 182; 202, 204), der eine Kammer (12; 37; 62; 90; 120b, 130; 120b, 144; 170; 120b, 184, 186; 120b, 208, 206) mit veränderlichem Volumen begrenzt,
- mit Einrichtungen (16; 36; 66; 88; 118) zum Verändern des Volumens der Kammer,
- mit einer Einlaßöffnung in Fluidverbindung mit der Kammer und
- mit einer Einrichtung (24; 54; 78; 98; 124; 146; 168; 188; 210), die quer über den Fluidweg durch die Einlaßöffnung angeordnet ist,
dadurch gekennzeichnet,
- daß die quer über den Fluidweg angeordnete Einrichtung eine fluiddurchlässige Membraneinrichtung aufweist,
- daß ein in einem Komplementbindungstest mit dem Probenbestandteil reaktionsfähiges Reagens innerhalb der Membraneinrichtung gehalten wird und
- daß die Kammer (12; 37; 62; 90, 120b, 130; 120b, 144; 170; 120b, 184, 186; 120b, 208, 206) gegenüber dem Außenraum des Behälters im wesentlichen fluiddicht ist, außer durch die Membraneinrichtung (24; 54; 78; 98; 124; 146; 168; 188; 210), wodurch eine Flüssigkeitsprobe auf der Membraneinrichtung außerhalb der Kammer (12; 37; 62; 90; 120b, 130; 120b, 144; 170; 120b, 184, 186; 120b, 208, 206) durch auf Betätigung der Volumenveränderungseinrichtung (16; 36; 66; 88; 118) hin erzeugtes Ansaugen durch die Membran in die Kammer gezogen wird, um das Volumen der Kammer zu vergrößern, und die Flüssigkeitsprobe innerhalb der Kammer unter auf Betätigung der Volumenveränderungseinrichtung (16; 36; 66; 88, 118) hin erzeugtem innerem Druck aus ihr heraus gedrückt wird, um das Volumen der Kammer zu verringern.

**2.** Testvorrichtung nach Anspruch 1, bei welcher die Volumenveränderungseinrichtung eine bewegliche Wandeinrichtung (16), die Teil der Behältereinrichtung ist, oder eine elastische, kollabierbare Wand (88) aufweist, die einen Teil der Kammer begrenzt und von der Außenseite der Vorrichtung zugänglich ist.

**3.** Testvorrichtung nach Anspruch 1, bei welcher die Kammer (12, 62) von einem hohlen Körper (14, 64) gebildet wird und innerhalb des Körpers ein Kolben (16, 66) für eine Bewegung in den Körper und aus ihm heraus auf eine relative Bewegung zwischen dem Kolben und dem Körper hin angebracht ist.

**4.** Testvorrichtung nach Anspruch 3, bei welcher der Kolben (16) für eine Bewegung in den Körper (14) und aus ihm heraus bei einer relativen Drehung zwischen dem Kolben und dem Körper mittels eines Gewindes angebracht (14b, 18e) ist, wobei der hohle Körper und die Kolbeneinrichtung vorzugsweise von außen zugängliche Greifoberflächen (14a, 18a) haben, durch die der Körper und der Kolben gefaßt und von Hand verdreht werden können, um das Volumen der Kammer zu verändern, oder in denen die Kolbeneinrichtung für eine Schiebebewegung in den Körper oder aus ihm heraus angebracht ist.

5. Testvorrichtung nach Anspruch 4, bei welcher der Kolben von dem hohlen Körper lösbar ist.

6. Testvorrichtung nach einem vorhergehenden Anspruch, bei welcher der Probenbestandteil ein Teil eines immunologischen Paares und das Reagens den anderen Teil des immunologischen Paares aufweist, wobei das Reagens vorzugsweise ein auf der Membraneinrichtung immobilisiertes Antigen oder einen auf der Membraneinrichtung immobilisierten Antikörper aufweist.

7. Testvorrichtung nach einem vorhergehenden Anspruch, welche einen Rand (18b) um die Membraneinrichtung herum aufweist, um die Flüssigkeitsprobe zu halten.

8. Testvorrichtung nach einem vorhergehenden Anspruch, hei welcher die Membraneinrichtung (24, 210) eine Membran (210a, 210b) und eine anliegende poröse Stützscheihe (24b; 210c) aufweist, wobei wahlweise ein trockenes, markiertes, in der Flüssigkeitsprobe lösliches Reagens auf der Stützscheibe angeordnet ist.

9. Testvorrichtung nach einem der Ansprüche 1 bis 7, bei welcher ein trockenes, markiertes, in der Flüssigkeitsprobe lösliches Reagens auf einer Oberfläche in Fluidverbindung mit der Kammer angeordnet ist.

10. Testvorrichtung nach einem vorhergehenden Anspruch, bei welcher die Kammer im wesentlichen frei von absorbierendem Material ist.

11. Testvorrichtung nach einem vorhergehenden Anspruch, hei welcher die Membraneinrichtung (54, 98; 124; 146) eine Oberfläche außerhalb der Kammer (37, 90) aufweist, die der Umgebung ausgesetzt oder von einer transparenten Wand (46, 48, 92) bedeckt ist.

12. Verfahren zur Feststellung von wenigstens einem vorbestimmten Bestandteil in einer Flüssigkeitsprobe, das
(a) das Kontaktieren der Flüssigkeitsprobe mit einer Seite einer fluiddurchlässigen Membraneinrichtung (24; 54; 78; 98; 124; 146; 168; 188; 210), die quer über einer Öffnung in eine ansonsten im wesentlichen gegenüber ihrer Umgebung abgedichteten Kammer (12; 37; 62; 90; 120b, 130; 120b, 144; 170; 120b, 184, 186; 120b, 208, 206) angeordnet ist, wobei die Kammer ein veränderliches Volumen hat, und
(b) das Verändern des Kammervolumens zur Bewegung der Flüssigkeitsprobe durch die Membraneinrichtung (24; 54; 73; 98; 124; 146; 168; 188; 210) in die Kammer (12; 37; 62; 90; 120b, 130; 120b; 144; 170; 120b, 184, 186; 120b, 208, 206) oder aus ihr heraus und wiederum das Verändern des Volumens der Kammer zur Bewegung der Flüssigkeitsprobe durch die Membraneinrichtung zurück aufweist, wodurch der vorbestimmte Bestandteil mit wenigstens einem Reagens in einem Komplementbindungstest reagieren kann, um auf der Memhraneinrichtung ein feststellbares Reaktionsprodukt als Anzeige dafür zu bilden, daß der Bestandteil in der Flüssigkeitsprobe vorhanden ist.

13. Verfahren nach Anspruch 12, bei welchem der vorbestimmte Bestandteil eine immunologische Substanz ist und das Reagens ein markiertes immunologisches Reagens aufweist.

14. Verfahren nach Anspruch 13, bei welchem das markierte immunologische Reagens ein Konjugat einer immunologischen Substanz mit einer Goldpartikelmarkierung aufweist.

15. Verfahren nach Anspruch 13 oder 14, bei welchem ein zweites Reagens vor einem Kontakt mit der Flüssigkeitsprobe auf der Membraneinrichtung immobilisiert wird, wobei das immobilisierte Reagens mit dem vorbestimmten Bestandteil immunologisch reaktionsfähig ist.

16. Verfahren nach Anspruch 15, hei welchem
(a) das markierte Reagens mit dem vorbestimmten Bestandteil, aber nicht mit dem immobilisierten Reagens immunologisch reaktionsfähig ist, so daß durch die Bindung des markierten Reagens an den vorbestimmten Bestandteil, der an das zweite Reagens gebunden wird, ein feststellbares sandwichartiges Reaktionsprodukt auf der Membraneinrichtung gebildet wird, oder

(b) das markierte Reagens vom gleichen immunologischen Typ ist wie der vorbestimmte Bestandteil und so mit dem immobilisierten Reagens, aber nicht mit dem markierten Reagens immunologisch reaktionsfähig ist, so daß ein Konkurrenzbindungstest auf der Membraneinrichtung stattfindet.

17. Verfahren nach einem der Ansprüche 13 bis 16, bei welchem das markierte Reagens in einer trockenen, löslichen Form auf einer der Kammer ausgesetzten Oberfläche der Vorrichtung abgeschieden wird, und bei seinem Durchgang durch die Membraneinrichtung in der Flüssigkeitsprobe gelöst wird.

18. Verfahren nach einem der Ansprüche 13 bis 17, bei welchem
(I) heim Schritt (a) die Flüssigkeitsprobe in der Kammer (12) abgeschieden und beim Schritt (b) durch Verringerung des Kammervolumens durch die Membraneinrichtung (24) aus der Kammer (12) herausbewegt wird oder
(II) heim Schritt (a) die Flüssigkeitsprobe zuerst auf der Außenseite der Membraneinrichtung (24) abgeschieden und beim Schritt (b) infolge einer Vergrößerung des Kammervolumens durch Saugen durch die Membraneinrichtung in die Kammer (12) gezogen wird.

19. Verfahren nach Anspruch 18 (I), hei welchem die Flüssigkeitsprobe an der äußeren Oberfläche der Membraneinrichtung gehalten wird.

20. Verfahren nach Anspruch 18 (I), das ferner den Schritt
(c) aufweist, danach das Kammervolumen zu vergrößern, um die Flüssigkeitsprobe von der äußeren Oberfläche der Memhraneinrichtung (24) zurück in die Kammer (12) zu ziehen.

21. Verfahren nach Anspruch 18 (II), das ferner den Schritt
(c) aufweist, danach das Kammervolumen zu verringern, um die Flüssigkeitsprobe durch die Memhraneinrichtung (24) zurück und aus der Kammer (12) heraus zu drücken.

22. Verfahren nach einem der Ansprüche 13 bis 21, bei welchem das Reagens auf wenigstens einem bestimmten Bereich auf der Membraneinrichtung konzentriert und das feststellbare Reaktionsprodukt an dem einen bestimmten Bereich gebildet wird und die Membraneinrichtung wahlweise ein zweites Reagens aufweist, das auf wenigstens einem im Abstand von dem ersten bestimmten Bereich angeordneten, zweiten bestimmten Bereich konzentriert wird, wobei das zweite Reagens mit einem zweiten vorbestimmten Bestandteil der Flüssigkeitsprobe, falls vorhanden, reaktionsfähig ist, um ein zweites feststellbares Reaktionsprodukt an dem zweiten bestimmten Bereich zu bilden.

FIG.—1

FIG.—2

FIG.—3A

FIG.—3B

FIG.—4

FIG.—5

FIG.—6

FIG.—7

FIG.—8

FIG.—9

FIG.—10

FIG.—11

FIG.—12

122a

122b

120

120b

122c

208

202

212

210

200

14

206

204

**FIG.—13**

210a

210b

210c

**FIG.—14**